# EUROPÄISCHE PATENTSCHRIFT

(11) **EP 0 346 611 B1**
(45) Veröffentlichungstag und Bekanntmachung des Hinweises auf die Patenterteilung: **29.12.1993**
(21) Anmeldenummer: 89108393.3
(22) Anmeldetag: 10.05.1989
(51) Int. Cl.: F04B 7/06, F04B 15/00

(54) **Verfahren zum Hitzesterilisieren von Pumpen**
Pump heatsterilisation process
Procédé de stérilisation par chaleur des pompes

(30) Priorität: 18.05.1988 DE 3816935
(43) Veröffentlichungstag der Anmeldung: 20.12.1989
(73) Patentinhaber: GRONINGER & CO. GMBH, D-74564 Crailsheim (DE)
(72) Erfinder: Groninger, Horst, D-7180 Crailsheim (DE)
(74) Vertreter: Kratzsch, Volkhard, Dipl.-Ing.

(56) Entgegenhaltungen:
- DE-U- 8 708 670
- US-A- 3 230 892
- US-A- 3 515 016

## Beschreibung

Die Erfindung betrifft ein Verfahren zum Hitzesterilisieren von Pumpen für flüssige oder pastöse Pharmazeutika, Kosmetika, Lebensmittel oder dergleichen, deren Kolben in einem Zylinder relativ zu diesem in seiner Längsrichtung im wesentlichen vertikal verschiebbar und um seine Achse verdrehbar antreibbar ist und eine bis zur kreiszylindrischen Zylinderwandung des Zylinders und bis zur Bodenfläche des Kolbens reichende Aussparung aufweist, die bei einem Saughub mit einem Saugstutzen in der Zylinderwandung und bei einem Druckhub mit einem Druckstutzen in der Zylinderwandung kommuniziert, wobei der Zylinder eine verschließbare Ablauföffnung aufweist.

Derartige Pumpen, die hauptsächlich in Vorrichtungen zum Abfüllen kosmetischer oder pharmazeutischer pastöser oder flüssiger Produkte verwendet werden, werden bisher im zerlegten Zustand im Autoklaven unter Druck hitzesterilisiert (Prospekt "12-stellige rundlaufende Kolbenfüllmaschine FKR C 12", Fa. Strunck, 2/83), woraus es für einen anderen Typ auch bekannt ist, den Zylinder der Pumpe in der Pumpvorrichtung eingebaut zu lassen, jedoch den Kolben vollständig nach oben aus dem Zylinder herauszuziehen (ihn freizustellen) und dann den Kolben und den Zylinder mit einem Sterilisationsmedium zu behandeln. Im letztgenannten Fall muß der Zylinder nach oben soweit mit vergrößerter Bohrung verlängert sein, daß er den freigestellten Kolben seitlich umgibt. Dadurch wird der Herstellungsaufwand vergrößert. In beiden Fällen ist die Sterilisation mit zusätzlichen aufwendigen Arbeiten verbunden. Insbesondere besteht die Möglichkeit, daß beim Zusammenbauen der sterilisierten Pumpe diese unsteril wird.

Der Erfindung liegt die Aufgabe zugrunde, die Hitzesterilisation zu vereinfachen. Diese Aufgabe wird gemäß der Erfindung durch die Merkmale im Kennzeichnungsteil des Anspruchs 1 gelöst.

Ein Vorteil besteht darin, daß der Pumpe das Sterilisationsmedium zugeführt werden kann, ohne daß die Pumpe vorher ausgebaut oder freigestellt werden muß. Die Pumpe wird vielmehr z.B. in der Abfüllvorrichtung auch beim Zuführen des Sterilisationsmediums z. B. in der gleichen Weise wie beim Pumpbetrieb angetrieben. Es hat sich überraschend gezeigt, daß bei Verwendung von heißem Wasserdampf als Sterilisationsmedium, eine zuverlässige Sterilisation der Pumpe im eingebauten Zustand erreicht werden kann. Die Pumpe wird in all ihren Teilen ausreichend stark erhitzt und dabei kommen die zu sterilisierenden Flächen ausreichend stark mit Feuchtigkeit in Kontakt. Bei der Erfindung ist weiter von Vorteil, daß auch mit der Pumpe verbundene Leitungen, die beim normalen Pumpbetrieb das zu fördernde Produkt führen, sterilisiert werden können, wenn das Sterilisationsmedium auch durch diese genannten Leitungen geleitet wird, was besonders einfach zu verwirklichen ist.

Das Sterilisationsmedium kann der Pumpe durch den Saugstutzen zugeführt und durch den Druckstutzen abgeführt werden. Besonders bei kleinen Pumpen mit einem Fördervolumen bis zu etwa 25 ml pro Hub hat es sich als günstig für eine rasche Erhitzung der Pumpe erwiesen, wenn das Sterilisationsmedium über eine im Boden des Zylinders vorhandene verschließbare Öffnung der Pumpe zugeführt wird. Die genannte Öffnung dient vorteilhaft auch dazu, mindestens nach Beendigung der Sterilisation, bei Bedarf aber auch während des Sterilisierens Flüssigkeit (Kondensat) aus der Pumpe abzuleiten.

Die Pumpe ist für die Anwendung des erfindungsgemäßen Sterilisierverfahrens besonders gut geeignet, wenn der Kolben und der Zylinder aus Keramik bestehen. Da Keramik eine äußerst geringe thermische Ausdehnung hat, besteht bei der Sterilisation unter dem Einfluß hoher Temperatur, z. B. bei Verwendung von Wasserdampf, nicht die Gefahr des Klemmens der Pumpe.

Es ist bekannt (DE-U-87 08 670), die Pumpen von Abfüllmaschinen zu reinigen, ohne daß ein Zerlegen der jeweiligen Pumpe erforderlich ist. Wird ein Reinigen der Pumpe notwendig, so wird dem Sauganschluß der Pumpe Reinigungsflüssigkeit zugeführt und eine Absperrvorrichtung geöffnet. Die Pumpe wird angetrieben, so daß die zum Reinigen dienende Flüssigkeit einerseits durch die Pumpe gefördert wird und andererseits durch einen im Boden des Zylinders vorgesehen Kanal abfließt. Die Reinigung erfolgt mit einer Flüssigkeit. Sie ermöglicht keine Sterilisation, d. h. Keimtötung, sondern lediglich ein Auswaschen von Medienpartikeln aus der Pumpe, ohne zu gewährleisten, daß etwaige Mikroorganismen einschließlich ihrer Dauerformen und Sporen abgetötet werden.

Vorteilhafte Weiterbildungen des Verfahrens gemaß der Erfindung ergeben sich aus den Ansprüchen 2 bis 6. Dadurch ergibt sich der Vorteil, daß der obere Endbereich des Kolbens und Zylinders vor dem Zutritt von nicht-steriler Umgebungsluft geschützt werden kann und dadurch das Eindringen von Keimen in die Pumpe zusätzlich erschwert wird.

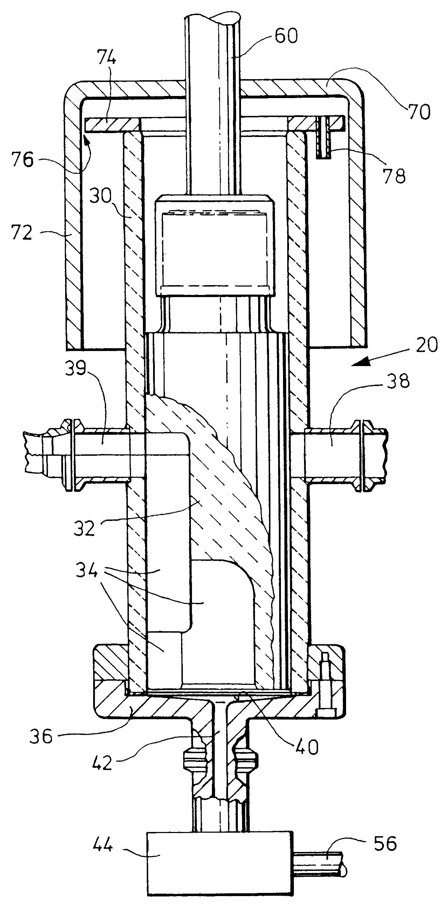

Ein Ausführungsbeispiel des Verfahrens gemäß der Erfindung ist nachfolgend anhand der Zeichnung näher erläutert, die einen Längsschnitt durch eine Pumpe zeigt.

In einer nicht gezeigten Abfüllmaschine für pastöse und flüssige Pharmazeutika ist eine Mehrzahl von Pumpen vorhanden. Eine derartige Pumpe 20 ist in der Figur gezeigt.

Die Pumpe 20 weist einen an einem Drehtisch der Maschine befestigten Zylinder 30 auf, der aus Keramik besteht und in dem ein Kolben 32, ebenfalls aus Keramik, in Längsrichtung des Zylinders, also vertikal, verschiebbar und gleichzeitig drehbar angeordnet ist. Der Kolben 32 weist in seinem oberen Bereich einen vollen, der inneren Kreisform des Zylinders 30 angepaßten Querschnitt auf, und er weist in seinem unteren Teil eine sich in Längsrichtung des Kolbens erstreckende und auf ihrer Länge sich zur Innenfläche des Zylinders 30 öffnende Aussparung 34 auf, die auch mit dem mittleren Bereich eines Bodens 36 des Zylinders, der mit dem Zylinder 30 fest verschraubt ist, in Verbindung ist. Etwa auf halber Höhe des Zylinders 30 sind in diesem diametral gegenüberliegend ein Saugstutzen 38 und ein Druckstutzen 39 vorgesehen. Die Funktion als Saugstutzen und Druckstutzen wird lediglich durch geeignete Kombination der Verschiebebewegung des Kolbens mit seiner Drehbewegung bewirkt.

Die Oberseite 40 des Bodens 36 fällt zu seiner Mitte hin ab, und der Boden 36 weist dort einen vertikal nach unten verlaufenden Kanal 42 auf, an dessen unteren Endbereich eine Absperrvorrichtung 44 angeschraubt ist. Diese stellt im offenen Zustand die Verbindung zwischen dem Kanal 42 und einem Ablaufrohr 56 her.

Das obere Ende des Kolbens 32 ist mit einer Kolbenstange 60 verbunden, durch die der Kolben in einer hin- und hergehenden Bewegung und gleichzeitig stets in der gleichen Richtung rotierend angetrieben wird. Ein hierfür geeigneter Antrieb ist in dem DE-GM 87 08 670 beschrieben und wird hiermit zum Gegenstand der vorliegenden Anmeldung gemacht.

Eine Glocke 70 verschließt das obere offene Ende des Zylinders 30. Die Glocke 70 ist an der Kolbenstange 60 befestigt und reicht mit einer zylindrischen Wand 72 in jeder Betriebsstellung des Kolbens 32 bis unter den Rand einer kreisringförmigen Scheibe 74, die am oberen Ende des Zylinders 30 befestigt ist. Der Innendurchmesser der Scheibe 74 ist etwas größer als der Innendurchmesser des Zylinders 30. Zwischen dem äußeren Rand der Scheibe 74 und der Wand besteht allseits ein Luftspalt 76. Durch einen Anschluß 78, der zum Anschließen eines Schlauchs vorgesehen ist, kann dem Inneren der Glocke 70 das Sterilisationsmedium unter einem kleinen Überdruck zugeführt werden, so daß dieses durch den Luftspalt 76 mit einer Stömungsgeschwindigkeit von etwa 0,45 m/s austritt und dadurch das Eindringen von Keimen in den Zylinder von oben her verhindert. Es ist daher kein Dichtring aus Kunststoff oder ähnlichem Material nötig. Die Glocke vergrößert nicht die Bauhöhe der Pumpe. Bei Bedarf kann ein weitgehend abgedichteter Raum, in den das obere Ende des Zylinders mündet, auch in anderer Weise verwirklicht werden. Die Glocke 70 und die Scheibe 74 sind im Beispiel aus Metall.

Im Abfüllbetrieb ist die Absperrvorrtchtung 44 im gesperrten Zustand. Während des Abfüllbetriebs fließt von einem zentral gelegenen Behälter das flüssige oder pastöse Abfüllgut zu dem Anschlußstutzen 38 der Pumpe und wird von dieser in Flaschen eingefüllt.

Wenn eine Reinigung (nicht Sterilisation) sämtlicher mit dem abzufüllenden Gut in Berührung kommender Teile der Abfülllmaschine erforderlich ist, so wird nach Entfernen des Abfüllguts dem Inneren des Vorratsbehälters für das Abfüllgut Reinigungsflüssigkeit zugeführt, und es wird nun diese beim weiteren Betrieb der Pumpe gefördert. Im Ausführungsbeispiel wird gleichzeitig die Absperrvorrichtung 44 geöffnet. Die Reinigungsflüssigkeit durchströmt nun, von dem Saugstutzen 38 der Pumpe kommend, den gesamten Innenraum der Pumpe, auch den Druckstutzen 39 und mit diesem verbundene weitere Teile und fließt auch durch den Kanal 42 im Boden 36 des Zylinders ab. Die Anordnung ist so getroffen, daß der Strömungswiderstand des Kanals 42 verhältnismäßig hoch ist, so daß beim Saughub der Pumpe nicht bereits verunreinigte Reinigungsflüssigkeit aus dem Kanal 42 in die Pumpe zurückgesaugt wird. Falls erforderlich, kann die Absperrvorrichtung als Ventil mit Fremdsteuerung ausgebildet werden und es kann jeweils beim Saughub der Pumpe die Absperrvorrichtung 44 geschlossen werden.

Beim Sterilisieren, dem das oben beschriebene Reinigen der Pumpe vorangehen kann, wird dem Inneren der Pumpe das Sterilisationsmedium zugeführt, im Beispiel ist dies Wasserdampf mit einer Temperatur von 130°C und einem Überdruck von 2 bar während einer Sterilisationszeit von etwa 20 Minuten.

Zum Sterilisieren muß das Sterilisationsmedium über einen Anschluß der Pumpe dem Innenraum der Pumpe zugeführt werden und über mindestens einen anderen Anschluß wieder aus dem Innenraum der Pumpe entfernt werden. Bei kleineren Pumpen bis maximal etwa 25 ml pro Hub hat es sich als vorteilhaft erwiesen, den Wasserdampf durch die zentrische Aussparung im Boden des Zylinders zuzuführen und durch den Druckstutzen und den Saugstutzen abzuleiten. Spätestens am Ende dar Sterilisation wird durch die Aussparung im Boden Kondenswasser abgeleitet.

Bei größeren Pumpen hat es sich dagegen als zweckmäßig erwiesen, den Wasserdampf durch den Saugstutzen zuzuführen und durch den Druckstutzen abzuleiten. Hier wird durch den zweckmäßigerweise im Boden vorhandenen Kanal das Kondenswasser dauernd abgeleitet. Alle Kanäle, durch die der heiße Wasserdampf strömt, werden sterilisiert. Daher ist es mit dem erfindungsgemäßen Verfahren auch möglich, Rohrleitungen, die zu der Pumpe und von der Pumpe wegführen, ebenfalls zu sterilisieren.

Während des Sterilisierens wird die Pumpe in gleicher Weise wie beim normalen Pumpbetrieb angetrieben. Dabei kommen alle Teile der Wandung im Inneren der Pumpe, die beim Pumpbetrieb mit dem zu pumpenden Medium in Kontakt kommen, auch mit dem heißen Wasserdampf in Kontakt. Darüberhinaus dringt der Wasserdampf auch in den äußerst engen Spalt zwischen dem Zylinder und dem Kolben ein.

Der Kolben und der Zylinder der Pumpe bestehen vorzugsweise aus Keramik mit sehr geringem Wärmeausdehnungskoeffizienten, damit nicht durch ungleichmäßige Erwärmung von Kolben und Zylinder ein Klemmen auftreten kann.

## Patentansprüche

1. Verfahren zum Hitzesterilisieren von Pumpen für flüssige oder pastöse Pharmazeutika, Kosmetika, Lebensmittel oder dergleichen, deren Kolben in einem Zylinder relativ zu diesem in seiner Längsrichtung im wesentlichen vertikal verschiebbar und um seine Achse verdrehbar antreibbar ist und eine bis zur kreiszylindrischen Zylinderwandung des Zylinders und bis zur Bodenfläche des Kolbens reichende Aussparung aufweist, die bei einem Saughub mit einem Saugstutzen in der Zylinderwandung und bei einem Druckhub mit einem Druckstutzen in der Zylinderwandung kommuniziert, wobei der Zylinder eine verschließbare Ablauföffnung aufweist,
**dadurch gekennzeichnet**,
daß die Pumpe während des Antriebs mittels eines heißen Sterilisationsmediums hitzesterilisiert wird,das einem Anschluß der Pumpe zugeführt wird.

2. Verfahren nach Anspruch 1,
**dadurch gekennzeichnet**,
daß der Kolben mit konstanter Drehrichtung rotierend angetrieben wird.

3. Verfahren nach Anspruch 1 oder 2,
**dadurch gekennzeichnet**,
daß der angetriebenen Pumpe heißer Wasserdampf als Sterilisationsmedium zugeführt wird.

4. Verfahren nach Anspruch 3,
**dadurch gekennzeichnet**,
daß der Wasserdampf mit einer Temperatur von 130°C und einem Überdruck von 2 bar zugeführt wird.

5. Verfahren nach einem der Ansprüche 1 bis 4,
**dadurch gekennzeichnet**,
daß das Sterilisationsmedium dem Inneren der Pumpe während einer Sterilisationszeit von etwa 20 Minuten zugeführt wird.

6. Verfahren nach einem der Ansprüche 1 bis 5,
**dadurch gekennzeichnet**,
daß einem zumindest annähernd abgedichteten Raum, in den das obere Ende des Zylinders mündet, über einen Anschluß (78) das Sterilisationsmedium zugeführt wird.

## Claims

1. Method for heat sterilizing pumps for liquid or pasty pharmaceutical products, cosmetics, foodstuffs or the like, whereof the piston is able to slide substantially vertically in its longitudinal direction in a cylinder relative to the latter and can be driven so that it rotates about its axis and comprises a recess extending as far as the circular cylindrical cylinder wall of the cylinder and as far as the bottom surface of the piston, which recess communicates with a suction connection in the cylinder wall at the time of a suction stroke and with a pressure connection in the cylinder wall at the time of a compression stroke, the cylinder comprising a closable discharge opening, characterised in that whilst being driven the pump is heat sterilised by means of a hot sterilisation medium, which is supplied to a connection of the pump.

2. Method according to Claim 1, characterised in that the piston is set in rotation with a constant direction of rotation.

3. Method according to Claim 1 or 2, characterised in that hot water vapour is supplied to the driven pump as the sterilisation medium.

4. Method according to Claim 3, characterised in that the water vapour is supplied at a temperature of 130°C and an excess pressure of 2 bars.

5. Method according to one of Claims 1 to 4, characterised in that the sterilisation medium is supplied to the inside of the pump for a sterilisation time of approximately 20 minutes.

6. Method according to one of Claims 1 to 5, characterised in that the sterilisation medium is supplied by way of a connection (78) to an at least approximately sealed chamber, into which the upper end of the cylinder opens.

## Revendications

1. Procédé de stérilisation à haute température de pompes pour des produits pharmaceutiques, cosmétiques, alimentaires, ou autres, liquides ou pâteux, dont le piston peut se déplacer dans un cylindre, par rapport à ce dernier, dans le sens de sa longueur et dans un sens essentiellement vertical, être entraîné en rotation autour de son axe, et présente un évidement, qui s'étend jusqu'à la paroi cylindrique circulaire du cylindre et jusqu'à la surface du fond du piston, cet évidement communiquant avec une tubulure d'aspiration prévue dans la paroi du cylindre, lors d'une course d'aspiration, et avec une tubulure de refoulement prévue dans la paroi du cylindre, lors d'une course de refoulement, et le cylindre présentant une ouverture de décharge obturable, caractérisé en ce que la pompe est stérilisée à haute température, pendant l'entraînement, au moyen d'un stérilisant envoyé par un raccord de la pompe.

2. Procédé suivant la revendication 1, caractérisé en ce que le piston est entraîné en rotation dans un sens constant.

3. Procédé suivant l'une des revendications 1 et 2, caractérisé en ce que la pompe entraînée est alimentée en vapeur d'eau surchauffée, comme stérilisant.

4. Procédé suivant la revendication 3, caractérisé en ce que la vapeur d'eau est envoyée à une température de 130°C et sous une pression effective de 2 bars.

5. Procédé suivant l'une quelconque des revendications 1 à 4, caractérisé en ce que le stérilisant est envoyé à l'intérieur de la pompe pendant une durée de stérilisation de l'ordre de 20 minutes.

6. Procédé suivant l'une quelconque des revendications 1 à 5, caractérisé en ce qu'un espace, rendu à peu près étanche du moins, et dans lequel débouche l'extrémité supérieure du cylindre, est alimenté en stérilisant par l'intermédiaire d'un raccord (78).
